# EUROPEAN PATENT SPECIFICATION

(11) **EP 1 919 435 B1**
(45) Date of publication and mention of the grant of the patent: **06.07.2011**
(21) Application number: 06789787.6
(22) Date of filing: 16.08.2006
(51) Int. Cl.: A61K 6/033, A61K 38/43

(54) **DENTAL COMPOSITION COMPRISING ENZYME**
DENTALZUSAMMENSETZUNG MIT EINEM ENZYM
COMPOSITION DENTAIRE COMPRENANT UNE ENZYME

(30) Priority: 17.08.2005 EP 05017845
(43) Date of publication of application: 14.05.2008
(73) Proprietor: 3M Innovative Properties Company, St. Paul, MN 55133-3427 (US)
(72) Inventor: KAPPLER, Oliver, 82229 Seefeld (DE); HAEBERLEIN, Ingo, R., 82229 Seefeld (DE)
(74) Representative: Brem, Roland
(86) International application number: PCT/US2006/031904
(87) International publication number: WO 2007/022219

(56) References cited:
- WO-A-2004/017988
- GB-A- 1 265 468
- US-A- 4 364 926
- US-A1- 2002 028 251
- US-A1- 2003 211 054
- US-A1- 2004 120 901
- DATABASE WPI Section Ch, Week 198241 Derwent Publications Ltd., London, GB; Class A96, AN 1982-86698E XP002366701 & JP 57 142910 A (SANKIN KOGYO KK) 3 September 1982 (1982-09-03)

## Description

### Field of Invention

The present invention relates to a dental composition. Furthermore, the present invention relates to a process of producing this dental composition. The invention also relates to the use of such dental compositions for producing a treatment agent for removing caries.

### Background

Caries, which is also called tooth decay, is one of the most frequently occuring human diseases. Caries is a bacterial damage of the tooth which may even cause teeth to fall out. From the outside, teeth are protected by a cover of hard enamel enclosing the softer dentin which in turn encloses the so-called pulp. The enamel itself consists of about 95 % inorganic compounds, especially hydroxyapatite, and about 5 % organic compounds and water. Dentin is softer than enamel and consists of about 65 % inorganic compounds (mainly hydroxyapatite), about 20 % organic compounds (mainly collagen and polysaccharides) and about 15 % water.

Caries develops in several steps by bacterial fermentation of carbohydrates, in particular by bacterial fermentation of sugar to acids. The acids resulting from said bacterial fermentation firstly dissolve the hard enamel, whereas bacteria attack mainly the organic components such as food particles having remained on the teeth.

If the enamel becomes porous and soft by the bacteria induced influence of acids, bacteria may reach the dentin layer below the enamel and infect it with caries. The region where the enamel or enamel and dentin are dissolved the most is called the caries lesion or cavity. The development of shallow cavities often continues and causes infection of the deeper parts of the tooth. These so called caries profunda situations show an extended area of caries infected dentin with the risk that the caries disease develops an inflammation of the pulp under the dentin. An inflammation of the pulp is extremely painful and may cause a serious risk to the health of the patient if it is not treated quickly.

The removal of the inflamed pulp through endodontic treatments includes disinfection of the root canal where the pulp is removed from. If this disinfection is not executed properly, the infection may persist and may even lead to infection of the alveolar bone.

Thus, the indicated treatment of a carious lesion is to remove the infected tissue and necrotic dentin.

It is well known that current caries excavation procedures do not remove all bacteria from the cavity (Kidd et al. 2003, in Fejerskov& Kidd, "Dental Caries", Blackwell Munksgaard, Chapter 17). Therefore, the use of cavity disinfectants is often recommended especially in deep caries lesion to prevent pulpal infections. (Brännström et al. 1980, Caries Res 14: 276-284). Prevention of pulpal inflammation is also the reason why prior to pulp capping, disinfection is indicated or why pulp-capping products were developed with antimicrobial properties.

Furthermore, disinfection is an integral part of endodontic therapy which is reflected in the statement that "endodontics is primarily a microbiological and then an anatomical and technical problem!" (Endodontie, Heinemann (ed.), Urban & Fischer 2001, p. 82). Especially problematic is the endodontic smear layer that is created during endodontic treatment and that hinders antibacterial substances from reaching the bacteria in the smear layer and in the tubuli of the canal walls. Today, endodontic therapy often is not successful in killing the bacteria in the smear layer and in the tubuli which may allow recolonization (Endodontie, Heinemann (ed.), Urban & Fischer 2001, p. 89). Furthermore, current endodontic irrigants may compromise endodontic sealers and restorations (Sevcigan et al., IADR 2005, poster #2982).

During treatment of a carious tooth, the dental practitioner may find different treatments are necessary: removal of necrotic dentin in shallower and deeper cavities and disinfection of the cavity. This disinfection of the cavity is especially advantageous in caries profunda situations where a pulpal infection is to be prevented. If the pulp is already infected it is necessary to remove the pulp and to disinfect and irrigate the endodontic region to prevent extensive infections.

The formulations disclosed in WO 2004/017988 remove infected dentin. They consist of at least one biologically active protease and/or at least one biologically active glycosidase. One embodiment of WO 2004/017988 contains water, acid, pepsin and a rheological additive.

EP 0884950 B1 and WO 00/27204 describe enzymes in combination with cationic polymers and N-hydroxyanilides, respectively, to kill bacteria.

US 2004/0071636 and WO 2004/000222 describe the use of bacteriophage derived lysozyme-like enzymes as anti-bacterial compounds.

### Summary

Applicants recognize that it would be desirable to have a product that specifically removes infected dentin and that contains agents to prevent bacterial contamination of the product.

This may be achieved by a dental composition as described in the claims comprising an enzyme that has an enzyme activity at acidic pH values. The composition typically contains a buffer that provides an acidic pH value and at least one preservative agent, wherein the at least one preservative agent is active at an acidic pH value.

### Brief Description of the Drawings

FIGURE 1 is a graph showing the antimicrobial action of various composition ingredients after 2 minutes.
FIGURE 2 is a graph showing the antimicrobial action of various composition ingredients after 5 minutes.

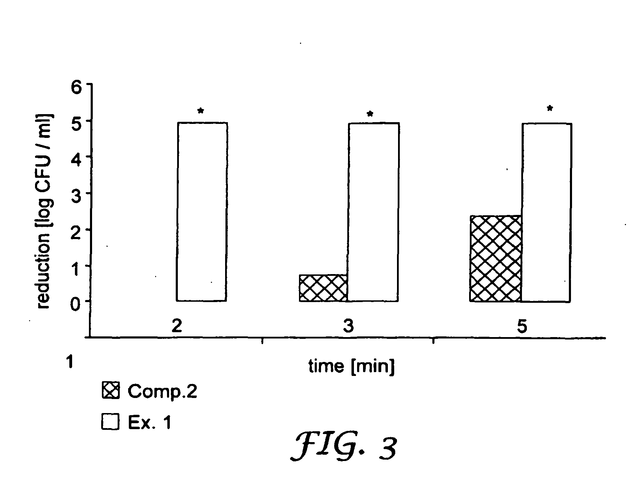

FIGURE 3 is a graph showing antimicrobial action of 2 compositions over time.

### Description

The presentation provides dental compositions comprising (i) an enzyme selected from the group of proteases having and enzyme activity at acidic pH levels, (ii) a buffer selected from phosphate buffer, carbonate buffer, acetate buffer, formate buffer, citrate buffer, tris buffer, glycylglycine buffer, glycine buffer or mixtures thereof providing an acidic pH value and (iii) at least one preservative agent selected from the group of benzoic acid an salts of benzoic acid. The enzyme activity of the enzyme used in the present invention typically does not have an optimum activity at the acidic pH value provided by the buffer. Nevertheless it preferably has a total enzyme activity at that pH value of at least 1 U/ml or more.

The composition according to the invention may have, for example, a total enzyme activity of from about 1 U to about 1,000,000 U per ml of composition. The lower end of the range of enzyme activity can be, for example, about 5 or 10 U/ml, wherein the inventive effect is normally considerably improved when the lower end of range of the total enzyme activity is about 20 or about 30 or about 50 U/ml composition.

Preferably, the total enzyme activity is about 500 to 50,000 U/ml of the composition or more preferably about 1,000 to about 8,000 U/ml of the composition. The desired activities can be obtained, for example, by adding in the case where pepsin is the enzyme, an amount of about 0.5 to about 15 mg (of ordinary, commercially available pepsin) per ml composition.

The enzyme unit (abbreviated as U) is the amount of enzyme necessary to convert one µmol of a corresponding enzyme substrate per minute at standard conditions for the respective enzyme. The enzyme units, as well as the standard conditions, are known in the art. For example, in WO 2004/017988, which is herein incorporated by reference, there are disclosed standard conditions for various enzymes.

In the special case of pepsin, for example, 1 Unit corresponds the amount of pepsin which releases 0.001 A280 as Trichloracetic acid soluble hydrolysis products from hemoglobin per minute at 37°C.

In the present case, the total enzyme activity [U/ml] relates to liquid as well as pasty and dry products which, for example, consist exclusively of a mixture of enzymes and optionally one or more adjuvants which are also dry.

The enzymes used in the present invention generally can be isolated from plants, animals or fungi as well as from bacteria or yeasts. They also may be produced by bioengineering. Representative examples of enzymes include those isolated from animals, bacteria or yeasts, such as enzymes obtained from pigs or chickens. Furthermore, those enzymes isolated from the bacterial genus *Streptomyces,* the fungus

*Penicillium,* the bacterial genus *Clostridium,* or the fungus *Aspergillus* or *Triotirachium* are suitable. Preferred enzymes are isolated from *Streptomyces griseus, Clostridium histolyticum, Alicyclobacillus, Aspergillus nidulans, penicillium species* or *Tritirachium album.*

In the context of the present invention, the term "proteases" denotes all enzymes capable of hydrolyzing peptide bonds. Furthermore, this term denotes enzymes capable of hydrolyzing dipeptides, oligopeptides, polypeptides and proteins or derivatives thereof such as glycosylated derivatives.

The proteases catalyze the decomposition of protein components which may be present in a caries lesion. For this purpose, generally any protease is suitable which catalyzes protein and peptide degradation. However, in the context of the present invention, especially preferred proteases are capable of catalyzing the decomposition of collagen fibers or of at least catalyzing a change in the structure of the collagen fibers so that the collagen fibers have better solubility characteristics after the protease reaction.

A classification of proteases suitable according to the invention is also possible with regard to the amino acids or co-factors involved in the proteolytic catalysis. Thus, in the context of the present invention, generally all protease classes such as serine proteases, matrix metalloproteases, aspartate proteases, serine-carboxyl proteinases and cysteine proteases may be used. In general, the proteases can be used under conditions which allow the respective proteases to catalyze the degradation of the protein components which are present in a caries lesion.

Compositions according to the invention may comprise one protease or two or more different proteases.

According to one embodiment of the present invention, the composition comprises, for example, one protease. According to another embodiment a composition according to the invention comprises about 1 to 10 different proteases, preferably 1 to 6, more preferably 1 to 4, even more preferably 2 to 3, and most preferably 2 different proteases.

According to another embodiment, the composition comprises only one protease, preferably an aspartate protease, wherein pepsin, and in particular pepsin from pigs, is preferred.

The enzyme comprising composition has a pH value in the acidic range, i.e., a pH value of less than 7.0.

Preferably the pH value of an inventive dental composition is in the range of about pH 1 to about pH 4.

In this respect, it is preferred that the enzyme has an enzyme activity at a pH value or a pH range of less than 4.0. It is not necessary that the enzyme has its maximum enzyme activity at this pH value, but it should have at least 10 % of its maximum activity at a pH value of less than 4.0. Consequently, the buffer should provide the dental composition with a pH value of less than 4.0.

The invention dental composition contains a buffer selected from phosphate buffer, carbonate buffer, acetate buffer, formate buffer, citrate buffer, tris buffer, glycylglycine buffer, glycine buffer or mixtures thereof. Sodium phosphate buffer, sodium hydrogen phosphate buffer, sodium dihydrogen phosphate buffer, potassium phosphate buffer, potassium hydrogen phosphate buffer, potassium dihydrogen phosphate buffer or pyrophosphate buffer are preferred. Suitable buffers also include sodium carbonate buffer, potassium carbonate buffer, sodium hydrogen carbonate buffer or potassium hydrogen carbonate buffer. Phosphate buffer and their components are especially preferred. An especially preferred phosphate buffer is a sodium dihydrogen phosphate buffer.

The buffer concentration preferably is in the range of about 0.01 to about 2.0 M.

The composition according to the present invention contains one or more preservatives. Generally, any type of preservative can be used that inhibits the growth of microorganisms and is tolerated by the human body. It has, however, been found that conventional preservatives of the para hydroxy benzoic acid ester type are preferred. Especially preferred are preservatives known under the names para hydroxy benzoic acid methyl ester (methylparabene) and para hydroxy benzoic acid propyl ester (propylparabene). Each of the named preservatives can be used as the sole preservative. It is, however, also possible to use combinations of such preservatives. The preservatives are generally used in an amount of about 0.001 to about 1 wt.-%, based on the weight of the composition. In a preferred embodiment of the present invention, preservatives are used in an amount of about 0.01 to about 0.25 wt.-%, based on the weight of the composition.

In addition to the optional preservatives, the composition according to the present invention requires at least one preservative agent that is active at an acidic pH value and preferably is active at an pH value of less than 4.0 , wherein The preservative agent is benzoic acid or salts of benzoic acid.

More preferably, the preservative agent is benzoic acid.

By adding a preservative agent active at acidic pH values to the dental composition a certain preservation is obtained. Thus, the composition shows anti-bacterial properties and is protected against bacterial contamination. Additionally to this preservative effect a surprisingly synergistic effect is reached by the Inventive composition. As known to a person skilled in the art the dental composition would according to the ingredients be useful to remove infected and/or necrotic dentin and/or an infected pulp. It would further be resistant against bacterial contamination. After testing the dental composition for its ability to reduce bacteria it was found that according to a synergistic effect the inventive composition reduces cariogenic bacteria by more than 5 log₁₀ levels in a very short time of treatment.

Thus, this composition is not only highly functional as a dental composition for caries removal or removal of pulp, pulpal residues, necrotic tissue and/or the endodontic smear layer in the root canal after endodontic treatment. It further exhibits protection against bacterial contamination. Additionally and due to its surprisingly effective antibacterial properties it can be used by the dental practitioner to disinfect the treated dental cavity after caries removal and/or to clean and/or disinfect a prepared tooth or an abutment. According to its biologically compatibility the inventive composition shows an advantage over the compositions for caries removal or disinfection of the cavity known from the art. It is further possible to use the dental composition according to the present invention as an irrigation fluid for the endodontic region or for endodontic treatment and/or to disinfect the root canal and/or the tubull of the canal walls after removal of the pulp. Therewith the endodontic smear layer is removed and the endodontic region is disinfected with one composition or in one step.

Particular advantageous is that the invention composition can be used for both steps explained above. Thus, the removal of necrotic tissue, carious tissue and infected dentin as well as removal of the pulp, pulpal residues and/or the endodontic smear layer in the root canal after endodontic treatment and the disinfection of the treated region is provided by only one composition. These treatments may be performed in only one step what is time saving over the treatment with compositions known from the art.

The composition according to the present invention may contain a rheological additive. As rheological additives, organic thickening agents are successfully used. These include starch, polyethylene glycol, polyvinylalcohol, polyvinyl pyrrolidone, xanthan, glycerin, hydroxyethyl propylcellulose, hydroxybutyl methylcellulose, hydroxypropyl methycellulose, hydroxyethylcellulose and sodium carboxymethylcellulose. Even possible are inorganic thickening agents such as silica gels or phyllosilicates. Mixtures of two or more of the mentioned thickening agents may be used.

It is preferred, when a protease containing composition according to the present invention contains a polysaccharide as a rheological additive. Suitable polysaccharides are, for example, starch, mannan, xanthan, alginate, carragen, pectin, polyvinyl pyrrolidone, polyvinylalcohol, hydroxyethyl propylcellulose, hydroxybutyl methylcellulose, hydroxypropyl methycellulose, hydroxyethylcellulose or sodium carboxymethylcellulose and mixtures of two or more thereof.

A solvent may be used in the present dental composition. In general, each solvent which is able to dissolve the enzymes and other components without denaturing the enzyme and/or without interfering with other components of the composition can be used. For example, any aqueous and organic solvent can be used that does not impair the activity of the enzyme. Suitable solvents include, for example, water, linear, branched or cyclic, saturated or unsaturated alcohols with 2 to about 10 C atoms, ketones, esters, carboxylic acids and mixtures of two or more of said types of solvents. Preferred solvents include water and water-alcohol mixtures.

Dialkyl ketones, alcohols or polymerizable substances of low viscosity such as polyethylene glycol (PEG), hydroxyethyl methacrylate or (2,3-epoxypropyl) methacrylate and mixtures thereof also can be used as solvents. Other suitable organic solvents include glycerin, dimethyl sulfoxide, tetrahydro furane, acetone, methyethyl ketone, cyclohexanol, toluene, methylen chloride, chloroform, alkanes and acetic acid alkyl esters, in particular acetic acid ethyl ester.

The composition can comprise additional adjuvants, for example such as those disclosed in WO 2004/017988 like complexing agents, enzyme substrates or enzyme effectors. According to the present invention, enzyme effectors comprise enzyme activators as well as enzyme inhibitors.

To enhance the storage properties of the composition, it can be advantageous to provide the inventive composition with two components, A and B, wherein component A comprises a solvent, an enzyme having an enzyme activity at acidic pH values and a buffer providing a pH value above the pH value at which the enzyme is most active.

Component A has a pH value within the range of 4.5 to 6.5. Preferably, it can be adjusted in a range of 5.0 to 6.0.

Component B comprises a solvent, a buffer system providing a pH value equal to or less than the pH value at which the enzyme is most active and a preservative agent active at said pH value.

This component B has a pH value within the range of 1.0 to 4.0. Preferably, it can be adjusted n a range of 1.5 to 3.5.

Thus, by the ability to choose different pH values for the components A and B some advantages are reached. The enzyme stability can be increased. It is further possible to add different preservative agents in the different components. Further, some additives show better solubility and/or efficiency at certain pH values. Thus, they can be added to that component A or B that has the more suitable pH value.

In one embodiment of the invention it is preferred that component A comprises water, an enzyme having an enzyme activity at acidic pH values, sodium phosphates and sodium hydroxide providing a pH value above the pH value at which the enzyme is most active, and a preservative agent. Component B comprises water, sodium phosphates and phosphoric acid providing a pH value equal to or less than the pH value at which the enzyme is most active, and a preservative agent active at said pH value.

In a preferred embodiment according to the present invention, component A can contain the following constituents in the following amounts:

| | |
|---|---|
| pepsin: | about 0.1 to about 1.0 wt.-% |
| sodium phosphates: | about 0.6 to about 2.4 wt.-% |
| sodium hydroxide: | about 0.001 to about 0.5 wt.-% |
| hydroxyethyl cellulose: | about 0.2 to about 1.0 wt.-% |
| methyl parabene: | about 0.005 to about 0.05 wt.-% |
| propyl parabene: | about 0.005 to about 1.0 wt.-% |
| water: | ad 100 wt.-% |

In a preferred embodiment according to the present invention, component B can contain the following constituents in the following amounts:

| | |
|---|---|
| sodium phosphates: | about 10 to about 20 wt.-% |
| phosphoric acid: | about 5 to about 10 wt.-% |
| polyethylene glycol: | about 2 to about 5 wt.-% |
| hydroxyethyl cellulose: | about 0.2 to about 1.0 wt.-% |
| sodium benzoate or benzoic acid: | about 0.01 to about 2.0 wt.-% |
| water: | ad 100 wt.-% |

The dental composition according to the present invention may be used to remove carious dentin and/or necrotic tissue. In another embodiment of the invention it may be used to remove the pulp, the endodontic smear layer and/or pulpal residues after endodontic treatment such as removal of the pulp and preparation of the root canal. The inventive composition has an advantageous biological compatibility.

It may be further used to disinfect the dental cavity and/or to clean and disinfect a prepared tooth or an abutment. This disinfection protects against reinfection of the cavity after the filling material is placed into the cavity and against a pulpal infection if the cavity is in an advanced state. The cleaning of the abutment after preparation of the tooth for prosthodontic purposes is advantageous as the smear layer and/or denatured collagenic residues are removed quickly.

It may be further used as an endodontic irrigation fluid and/or to disinfect the root canal, the tubuli of the canal walls and/or the endodontic region.

### Examples:

Microbial disinfection procedures are considered effective when during the given treatment time more than 99.999% of the originally existing microorganisms are inactivated which corresponds to 5 log₁₀ levels (DGHM, List of Disinfectants, 2003). Thus, a caries removal system that, within the given treatment time or faster, reduces cariogenic bacteria by more than 5 log₁₀ levels is highly functional.

The antimicrobial action of the present invention was evaluated with acidogenic strains of the cariogenic genera Streptococcus and Lactobacillus. An experimental system was developed which allows the detection of a reduction in bacterial levels of more than 5 log₁₀ levels. Streptococcus mutans DSM20523 and Lactobacillus paracaseii DSM 5622 were obtained from DSMZ, Braunschweig and stock cultures were generated by plating on Columbia/sheep blood-Agar (Oxoid) and Rogosa-Agar (Oxoid) respectively. Single colonies were used to inoculate 10 ml of Brain-Heart-Infusion (BHI) which was incubated at 37°C. 1-2 % of the 10 ml culture were used to inoculate 100 ml which was grown until stationary phase and concentrated by centrifugation.

Experiments were started by addition of 100 µl of concentrated bacterial suspension (S. mutans or L. paracasei) to 900 µl of the respective solution according to the examples. With the addition of the respective solution to the bacterial suspension the inactivation reaction started. The mixtures were equilibrated to 37°C in a thermostat. After the time intervals indicated samples of 100 µl were removed and mixed with nutrient broth to terminate the inactivation reaction. Control experiments showed that nutrient broth effectively terminated the inactivation reaction because no further decrease in Colony Forming Units ( = CFU) occurred.

Bacterial concentrations were determined as CFU by plating appropriate dilutions on agar plates.

The efficiency of the respective solution was measured by the reduction of CFU for each experiment. The reduction of bacteria is denoted in log₁₀ reductions. Log reductions were calculated by log₁₀ (CFU+1)tₒ - log₁₀ (CFU+1)tₓ (Paddick et al. 2003, Appl. Environ Microbiol 71: 2467-2472).

For each experiment to represents the sample at the starting time (t=0) and tₓ the sample at a given time point. This is depicted on the Y-axis of all graphs as "reduction (log₁₀ CFU /ml)".

### Example 1:

An aqueous solution A was prepared by mixing:

| Ingredient | concentration [weight-%] |
|---|---|
| Sodium phosphates | 1.5 |
| Sodium hydroxide | < 0.1 % |
| Hydroxyethyl cellulose | 0.5 |
| Pepsin | 0.9 |
| methyl parabene | 0.07 |
| propyl parabene | 0.02 |
| Water | Ad 100 |

All ingredients except of sodium hydroxide were mixed together and the sodium hydroxide was than added drop by drop to adjust the solution A to a pH value of 5.5.

An aqueous solution B was prepared by mixing:

| ingredient | concentration [weight-%] |
|---|---|
| Sodium phosphates | 15 |
| Phosphoric acid | < 6 |
| Polyethylene glycol 200 | 4 |
| Hydroxyethyl cellulose | 0.5 |
| Sodium benzoate | 0.12 |
| Water | Ad 100 |

All ingredients except of phosphoric acid were mixed together and the phosphoric acid was than added slowly to adjust the solution B to a pH value of 2.1.

Both solutions A and B were mixed at a ratio of A:B of 1:3. The resulting dental composition (Ex.1) was used as follows in the examples 2, 3 and 4.

### Comparative Example 1

A comparative example (Comp.1) was prepared as follows:

An aqueous solution A' was prepared by mixing

| ingredient | concentration [weight-%] |
|---|---|
| Sodium phosphate | 1.5 |
| Sodium hydroxide | < 0.1 % |
| Water | Ad 100 |

All ingredients except of sodium hydroxide were mixed together and the sodium hydroxide was then added drop by drop to adjust the solution A' to a pH value of 5.5.

The solution B' was prepared by mixing:

| ingredient | concentration [weight-%] |
|---|---|
| Sodium phosphate | 15 |
| Phosphoric acid | < 6 |
| Water | Ad 100 |

All ingredients except of phosphoric acid were mixed together and the phosphoric acid was then added slowly to adjust the solution B' to a pH value of 2.1.

Both solutions A' and B' were mixed at a ratio of A':B' of 1:3. The resulting comparative composition (Comp.1) was used as follows in the example 2, 3.

### Example 2:

To show the antimicrobial action of Ex.1 and Comp.1 against cariogenic bacteria the following Example 2 was performed. Both solutions were added to concentrated bacterial suspension as explained above.

Values are shown as means (SD) where bacteria were detectable after the treatment. Values marked with ">" represent experiments where no bacteria could be cultivated after the treatment. Thus, the values marked "> 5" represent experiments where the killing reproducibly exceeded 5-log₁₀ units. "N/D" means not determined.

| Log₁₀-reduction of Ex.1 and Comp.1 against cariogenic bacteria [log₁₀ CFU/ml] | | | | |
|---|---|---|---|---|
| | L. paracasei | | S.mutans | |
| t[min] | Ex.1 | Comp.1 | Ex.1 | Comp.1 |
| 0.5 | 1.6 (0.32) | 0.25 (0.35) | N/D | N/D |
| 1 | > 5 | -0.07 (0.51) | > 4.8 | -0.10 (0.13) |
| 2 | > 5 | 0.27 (0.33) | > 5 | 0.27 (0.57) |
| 3 | N/D | N/D | > 5 | 0.65 (0.49) |
| 5 | > 5 | 1.46 (1.00) | > 5 | 1.25 (0.69) |

As can be seen from the data obtained here Ex.1 shows a strong bacterial reduction already after 1 minute. To the contrary the Comp.1 exhibits no bacterial reduction after 1 min and only a small reduction within the given time period of 5 minutes.

### Example 3:

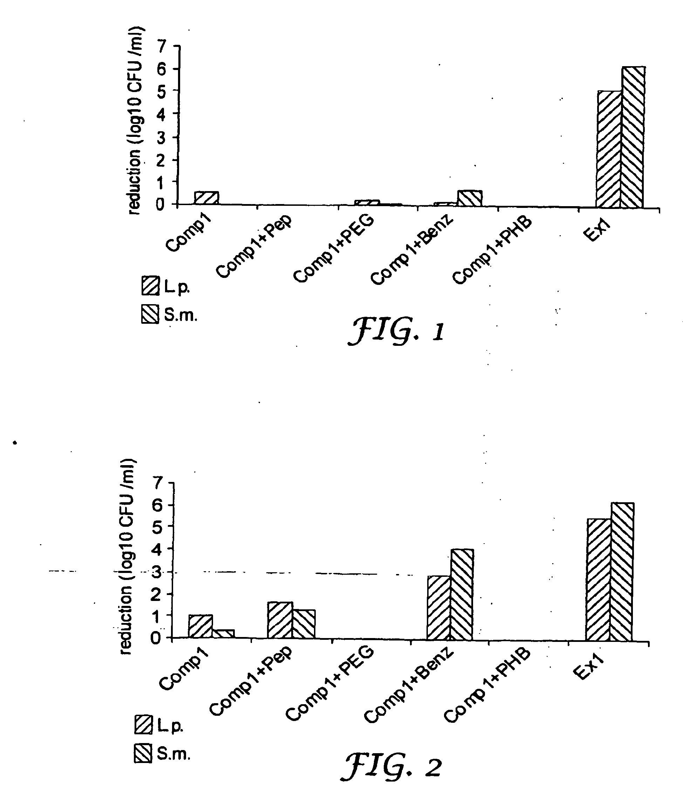

The antimicrobial action of the single ingredients of Ex. 1 was determined separately for the following substances after 2 min FIG. 1 and after 5 min FIG. 2:
Pep = Pepsin
PEG = Polyethylene glycol 200
Benz = Sodium benzoate
PHB = methyl/propyl parabenes

Thus, mixtures were prepared containing the ingredients of Comp.1 together with one of the indicated substances (Pep, PEG, Benz, PHB). The concentrations of each of the indicated substances were equal to those in Ex.1. The concentration of water according to Comp.1 was decreased accordingly.

In all experiments with a reduction greater than 5 log₁₀ units the limit of detection was reached. The log reduction is therefore greater than the value depicted.

It can be seen from the results that after 2 minutes as well as after 5 minutes the antimicrobial action cannot be attributed to any single ingredient, rather the synergistic action of the formulation is the basis of the strong antibacterial effect.

### Comparative Example 2:

A comparative example (Comp.2) was prepared as follows:

An aqueous solution A" was prepared by mixing:

| ingredient | concentration [weight-%] |
|---|---|
| Sodium phosphate | 1.5 |
| Sodium hydroxide | < 0.1 % |
| Hydroxyethyl cellulose | 0.5 |
| Pepsin | 0.9 |
| methyl parabene | 0.07 |
| propyl parabene | 0.02 |
| Water | Ad 100 |

All ingredients except of sodium hydroxide were mixed together and the sodium hydroxide was then added drop by drop to adjust the solution A" to a pH value of 5.5.

The solution B" was prepared by mixing:

| ingredient | concentration [weight-%] |
|---|---|
| Sodium phosphate | 15 |
| Phosphoric acid | < 6 |
| Polyethylene glycol 200 | 4 |
| Hydroxyethyl cellulose | 0.5 |
| Water | Ad 100 |

All ingredients except of phosphoric acid were mixed together and the phosphoric acid was then added slowly to adjust the solution B" to a pH value of 2.0.

Both solutions A" and B" were mixed at a ratio of A":B" of 1:3. The resulting comparative composition (Comp.2) was used as follows in the example 4.

### Example 4:

Ex.1 was tested against Comp.2 as disclosed in WO 2004/017988. In all experiments marked with * the limit of detection was reached. The log₁₀ reduction is therefore greater than the value depicted.

## Claims

1. A dental composition comprising an enzyme selected from the group of proteases having an enzyme activity at acidic pH values, a buffer selected from phosphate buffer, carbonate buffer, acetate buffer, formate buffer, citrate buffer, tris buffer, glycylglycine buffer, glycine buffer or mixtures thereof providing an acidic pH value and one or more preservative agents, wherein at least one preservative agent is selected from the group consisting of benzoic acid or salts of benzoic acid, wherein the pH value of the composition is less than 7.0.

2. The dental composition according to claim 1, wherein the enzyme has an enzyme activity at a pH value or a pH range of less than 4.0 and the buffer provides said pH value or said pH range.

3. The dental composition according to any of the preceding claims, wherein the buffer concentration is in the range of 0.01 to 2.0 M.

4. The dental composition according to any of the preceding claims, wherein the enzyme is pepsin.

5. The dental composition according to any of the preceding claims additionally containing a rheological additive and/or a solvent.

6. The dental composition according to any of the preceding claims comprising sodium phosphates, phosphoric acid, sodium hydroxide, polyethylene glycol 200, hydroxethyl cellulose, pepsin, sodium benzoate, p-hydroxybenzoic acid methyl ester, p-hydroxybenzoic acid propyl ester and water.

7. The dental composition according to any of the preceding claims comprising a para hydroxy benzoic acid ester.

8. A. dental composition comprising two components A and B, wherein component A comprises a solvent, an enzyme selected from the group of proteases having an enzyme activity at acidic pH values and a buffer selected from phosphate buffer, carbonate buffer, acetate buffer, formate buffer, citrate buffer, tris buffer, glycylglycine buffer, glycine buffer or mixtures thereof providing a pH value above the pH value at which the enzyme is most active; and
component B comprises a solvent, a buffer providing a pH value equal to or less than the pH value at which the enzyme is most active and a preservative agent wherein at least one preservative agent is selected from the group consisting of benzoic acid or salts of benzoic acid.
wherein the pH value of the composition is less than 7.0, wherein the buffer comprised in component A provides a pH value of 4.5 to 6.5 and the buffer comprised in component B provides a pH value of 1.0 to 4.0.

9. The dental composition according to claim 8 , wherein
component A comprises water, an enzyme having an enzyme activity at acidic pH values, sodium phosphates and sodium hydroxide as the buffer and a preservative agent; and
component B comprises water, sodium phosphates and phosphoric acid as the buffer and a preservative agent wherein at least one preservative agent is selected from the group consisting of benzoic acid or salts of benzoic acid.

10. The dental composition according to any of the preceding claims for use to remove carious dentin and/or to disinfect the dental cavity.

11. The dental composition according to any of the preceding claims for use as an endodontic irrigation fluid and/or to disinfect the root canal.

12. The dental composition according to any of the preceding claims for use to clean and/or disinfect a prepared tooth or an abutment.

## Patentansprüche

1. Dentalzusammensetzung, umfassend ein Enzym aus der Gruppe der Proteasen mit Enzymaktivität bei sauren pH-Werten, einen unter Phosphatpuffer, Carbonatpuffer, Acetatpuffer, Formiatpuffer, Citratpuffer, Tris-Puffer, Glycylglycinpuffer, Glycinpuffer oder Mischungen davon ausgewählten Puffer, der einen sauren pH-Wert bereitstellt, und ein oder mehrere Konservierungsmittel, wobei das mindestens eine Konservierungsmittel aus der Gruppe bestehend aus Benzoesäure oder Salzen von Benzoesäure ausgewählt ist, wobei der pH-Wert der Zusammensetzung weniger als 7,0 beträgt.

2. Dentalzusammensetzung nach Anspruch 1, wobei das Enzym bei einem pH-Wert oder in einem pH-Bereich von weniger als 4,0 Enzymaktivität aufweist und der Puffer diesen pH-Wert bzw. pH-Bereich bereitstellt.

3. Dentalzusammensetzung nach einem der vorhergehenden Ansprüche, wobei die Pufferkonzentration im Bereich von 0,01 bis 2,0 M liegt.

4. Dentalzusammensetzung nach einem der vorhergehenden Ansprüche, wobei es sich bei dem Enzym um Pepsin handelt.

5. Dentalzusammensetzung nach einem der vorhergehenden Ansprüche, die zusätzlich ein Rheologieadditiv und/oder ein Lösungsmittel enthält.

6. Dentalzusammensetzung nach einem der vorhergehenden Ansprüche, umfassend Natriumphosphate, Phosphorsäure, Natriumhydroxid, Polyethylenglykol 200, Hydroxyethylcellulose, Pepsin, Natriumbenzoat, p-Hydroxybenzoesäuremethylester, p-Hydroxybenzoesäurepropylester und Wasser.

7. Dentalzusammensetzung nach einem der vorhergehenden Ansprüche, umfassend einen para-Hydroxybenzoesäureester.

8. Dentalzusammensetzung, umfassend zwei Komponenten A und B, wobei Komponente A ein Lösungsmittel, ein Enzym aus der Gruppe der Proteasen mit Enzymaktivität bei sauren pH-Werten, einen unter Phosphatpuffer, Carbonatpuffer, Acetatpuffer, Formiatpuffer, Citratpuffer, Tris-Puffer, Glycylglycinpuffer, Glycinpuffer oder Mischungen davon ausgewählten Puffer, der einen pH-Wert bereitstellt, der über dem pH-Wert liegt, bei dem das Enzym am aktivsten ist, umfaßt und
Komponente B ein Lösungsmittel, einen Puffer, der einen pH-Wert bereitstellt, der kleiner oder gleich dem pH-Wert ist, bei dem das Enzym am aktivsten ist, und ein Konservierungsmittel umfaßt, wobei das mindestens eine Konservierungsmittel aus der Gruppe bestehend aus Benzoesäure oder Salzen von Benzoesäure ausgewählt ist, wobei der pH-Wert der Masse weniger als 7,0 beträgt, wobei der in Komponente A enthaltende Puffer einen pH-Wert von 4,5 bis 6,5 bereitstellt und der in Komponente B enthaltende Puffer einen pH-Wert von 0 bis 4,0 bereitstellt.

9. Dentalzusammensetzung nach Anspruch 8, wobei
Komponente A Wasser, ein Enzym mit Enzymaktivität bei sauren pH-Werten, Natriumphosphate und Natriumhydroxid als den Puffer und ein Konservierungsmittel umfaßt und
Komponente B Wasser, Natriumphosphate und Phosphorsäure als den Puffer und ein Konservierungsmittel umfaßt, wobei das mindestens eine Konservierungsmittel aus der Gruppe bestehend aus Benzoesäure oder Salzen von Benzoesäure ausgewählt ist.

10. Dentalzusammensetzung nach einem der vorhergehenden Ansprüche zur Verwendung zur Entfernung von kariösem Dentin und/oder zur Desinfektion der Dentalkavität.

11. Dentalzusammensetzung nach einem der vorhergehenden Ansprüche zur Verwendung als endodontische Spülflüssigkeit und/oder zur Desinfektion des Wurzelkanals.

12. Dentalzusammensetzung nach einem der vorhergehenden Ansprüche zur Verwendung zur Reinigung und/oder Desinfektion eines vorbereiteten Zahns oder eines Abutments.

## Revendications

1. Composition dentaire comprenant une enzyme choisie dans le groupe des protéases ayant une activité enzymatique à des valeurs de pH acide, un tampon choisi parmi un tampon phosphate, un tampon carbonate, un tampon acétate, un tampon formiate, un tampon citrate, un tampon tris, un tampon glycylglycine, un tampon glycine ou des mélanges de ceux-ci, assurant une valeur de pH acide et un ou plusieurs agents conservateurs, au moins un agent conservateur étant choisi dans le groupe constitué de l'acide benzoïque ou des sels d'acide benzoïque, la valeur de pH de la composition étant inférieure à 7,0.

2. Composition dentaire selon la revendication 1, **caractérisée en ce que** l'enzyme a une activité enzymatique à une valeur de pH ou dans une gamme de pH inférieure à 4,0 et le tampon assure ladite valeur de pH ou ladite gamme de pH.

3. Composition dentaire selon l'une ou l'autre des revendications précédentes, **caractérisée en ce que** la concentration de tampon est dans la gamme de 0,01 à 2,0 M.

4. Composition dentaire selon l'une quelconque des revendications précédentes, **caractérisée en ce que** l'enzyme est la pepsine.

5. Composition dentaire selon l'une quelconque des revendications précédentes, contenant en outre un additif rhéologique et/ou un solvant.

6. Composition dentaire selon l'une quelconque des revendications précédentes, comprenant des phosphates de sodium, de l'acide phosphorique, de l'hydroxyde de sodium, du polyéthylèneglycol 200, de l'hydroxyéthylcellulose, de la pepsine, du benzoate de sodium, de l'ester méthylique d'acide p-hydroxybenzoïque, de l'ester propylique d'acide p-hydroxybenzoïque et de l'eau.

7. Composition dentaire selon l'une quelconque des revendications précédentes, comprenant un ester d'acide para-hydroxybenzoïque.

8. Composition dentaire comprenant deux composants A et B, **caractérisée en ce que**
le composant A comprend un solvant, une enzyme choisie dans le groupe des protéases ayant une activité enzymatique à des valeurs de pH acide et un tampon choisi parmi un tampon phosphate, un tampon carbonate, un tampon acétate, un tampon formiate, un tampon citrate, un tampon tris, un tampon glycylglycine, un tampon glycine ou des mélanges de ceux-ci, assurant une valeur de pH supérieure à la valeur de pH à laquelle l'enzyme est la plus active ; et
le composant B comprend un solvant, un tampon assurant une valeur de pH inférieure ou égale à la valeur de pH à laquelle l'enzyme est la plus active et un agent conservateur, au moins un agent conservateur étant choisi dans le groupe constitué de l'acide benzoïque ou des sels d'acide benzoïque, la valeur de pH de la composition étant inférieure à 7,0, le tampon contenu dans le composant A assurant une valeur de pH de 4,5 à 6,5 et le tampon contenu dans le composant B assurant une valeur de pH de 1,0 à 4,0.

9. Composition dentaire selon la revendication 8, **caractérisée en ce que** le composant A comprend de l'eau, une enzyme ayant une activité enzymatique à des valeurs de pH acide, des phosphates de sodium et de l'hydroxyde de sodium en tant que tampon et un agent conservateur ; et
le composant B comprend de l'eau, des phosphates de sodium et de l'acide phosphorique en tant que tampon et un agent conservateur, au moins un agent conservateur étant choisi dans le groupe constitué de l'acide benzoïque ou des sels d'acide benzoïque.

10. Composition dentaire selon l'une quelconque des revendications précédentes, destinée à être utilisée pour enlever la dentine cariée et/ou pour désinfecter la cavité dentaire.

11. Composition dentaire selon l'une quelconque des revendications précédentes, destinée à être utilisée comme liquide d'irrigation endodentique et/ou pour désinfecter le canal des racines.

12. Composition dentaire selon l'une quelconque des revendications précédentes, destinée à être utilisée pour nettoyer et/ou désinfecter une dent préparée ou un pilier.
